# EUROPEAN PATENT APPLICATION

(11) **EP 3 398 519 A1**
(43) Date of publication of application: **07.11.2018**
(21) Application number: 17168978.9
(22) Date of filing: 02.05.2017
(51) Int. Cl.: A61B 8/00

(54) **DETERMINING A GUIDANCE SIGNAL AND A SYSTEM FOR PROVIDING A GUIDANCE FOR AN ULTRASONIC HANDHELD TRANSDUCER**

(71) Applicant: Koninklijke Philips N.V., 5656 AE Eindhoven (NL)
(72) Inventor: SENEGAS, Julien, 5656 AE Eindhoven (NL); LORENZ, Cristian, 5656 AE Eindhoven (NL); WISCHMANN, Hans-Aloys, 5656 AE Eindhoven (NL); KRUEGER, Sascha, 5656 AE Eindhoven (NL)
(74) Representative: de Haan, Poul Erik

(57) **Abstract**

The present invention relates the device and the method for providing a guidance signal. The device preferably relates to a mobile device, such as a mobile tablet computer. The device comprises an input unit, a display and a processing unit. Via the input unit, a three-dimensional outline image of a surface of a human subject is provided, e.g. acquired by a camera. The device further comprises a memory. The memory stores a human reference model, which statistically represents a virtual human subject. In practice it is often the case that the surface outline represented by the human reference model would not instantly fit to the surface outline of the human subject. Therefore, the processing unit is configured to adapt the human reference model resulting in an adapted model, such that the surface outline represented by the adapted model fits to the surface outline of the (real) human subject. Furthermore, the image is acquired as a so-called track-image of an ultrasonic handheld transducer in front of the human subject. The processing unit is configured to recognize the ultrasonic handheld transducer in the track-image and to determine the transducer pose based thereon. In practice, the ultrasonic handheld transducer is to be arranged on the surface of the human subject at a target pose in order to scan a desired scan region of the human subject. The scan region may relate to an inner organ of the human subject. Based on the actual transducer pose and the desired target pose, the processing unit is configured to determine a guidance signal, which indicates how to move and/or rotate the ultrasonic handheld transducer to reach the desired target pose.

## Description

### FIELD OF THE INVENTION

The present invention relates to a device and a method for determining a guidance signal. The invention further relates to a system for providing a guidance for an ultrasonic handheld transducer. Moreover, the present invention relates to a computer program element and a computer-readable medium.

### BACKGROUND OF THE INVENTION

Ultrasonic handheld transducers are known and are used in diagnostic imaging technique based on the application of ultrasonic. The term ultrasonic may also be referred to as ultrasound. Ultrasonic handheld transducers may be used for medical investigations for diagnostics, disease characterization and/or intra-operative guiding and/or imaging. An ultrasonic handheld transducer may be used as a "handheld" device or mobile device. Thus, it can be positioned and/or oriented very flexible and being arranged in an arbitrary position and/or orientation with respect to a human subject. Ultrasonic imaging via an ultrasonic handheld transducer is cost-effective, mobile and already available. The value of an ultrasonic image acquired via an ultrasonic handheld transducer often depends on the operator skills handling the ultrasonic handheld transducer. In particular, a navigation of the ultrasonic handheld transducer to an optimal transducer position and/or to an optimal transducer orientation to acquire an ultrasonic image for a given screening and/or diagnostic task is often difficult. As a consequence, often only expert medical staff, such as radiologists, sonographers, cardiologists and/or other trained physicians, may perform a suitable ultrasonic examination via an ultrasonic handheld transducer.

US 2013/0237811 A1 relates to a method and a system for tracking and guiding sensors and instruments. Said document discloses an ultrasonic transducer showing a housing with a machine-vision camera system. The integrated camera views an object, such as a patient's body, and determines the ultrasonic transducer's x, y, z position in space and pitch, yaw and roll orientation with respect to the object. The position and orientation at a point in time are saved along with an ultrasonic scan at the same point of time in a record file as a spatial register scan. Then, spatially registered scans of the same region of the body are compared in order to reduce ultrasonic artefacts in speckles, and tissue types and elastomeric properties can be refined. A three-dimensional model of tissue can be shown to a user. Further, a spatial registration apparatus is disclosed, which includes a memory having instructions for execution by at least one processor configured to determine a spatial position and orientation of the ultrasonic transducer with respect to an object using an image captured by the camera. Further, a display can be operatively connected with the processor, the display is configured for displaying a three-dimensional representation of the object created or refined from the determined spatial position and orientation and output from the ultrasonic transducer. The display can also be used for displaying a location of an item of interest or a saved position and orientation of a sensor probe with respect to the medical instrument. The displaying may include a graphical guiding element, such as a directional arrow.

Even though the previously discussed document may provide graphical guiding elements and to use multiple spatially registered scans of the same region of the body for comparing a purpose and for reducing ultrasonic artefacts in speckles, the issue remains to provide reliable ultrasonic images, if an ultrasonic handheld transducer is not operated by a medical expert.

### SUMMARY OF THE INVENTION

There may be a need for an automated guidance allowing an unexperienced operator to position and/or orient an ultrasonic handheld transducer with respect to a human subject, such that a reliable ultrasonic image may be acquired via the ultrasonic handheld transducer.

The object of the present invention is solved by the subject-matter of each of the independent claims. Further embodiments are incorporated in the respective dependent claims. It should be noted that the following described aspects of the present invention may apply at least in an analogous matter also for the device, the system, the method, the computer program element and the computer-readable medium.

According to a first aspect of the present invention, a device for determining a guidance signal is provided. The device comprises a input unit and a processing unit. The input unit is configured to receive an at least indirectly acquired outline (8) image of a surface outline of a human subject. The processing unit is configured to access a human reference model. The human reference model represents a surface outline of a virtual human subject, its internal morphology and a relation between its surface outline and its internal morphology. The processing unit is further configured to adapt the human reference model resulting in an adapted model, such that the surface outline represented by the adapted model fits to the imaged surface outline of the human subject. The processing unit is further configured to access a transducer model, which represents a surface outline of an ultrasonic handheld transducer and a detection range (field of view) of a probe of the ultrasonic handheld transducer. The input unit is configured to receive the image acquired as a track-image of the ultrasonic handheld transducer and a surrounding region of the surface of the human subject surrounding the ultrasonic handheld transducer, when the ultrasonic handheld transducer is arranged on the surface of the human subject. The processing unit is further configured to recognize the ultrasonic handheld transducer in the track-image based on the transducer model deriving in a transducer pose (transducer orientation) of the ultrasonic handheld transducer with respect to the human subject. The processing unit is configured to receive a target signal representing at least indirectly a scan region of the internal morphology of the adapted model. The processing unit is configured to determine a target pose for the ultrasonic handheld transducer with respect to the human subject based on the target signal, the transducer model and the adapted model resulting in a virtual match of the detection range and the scan region. The processing unit is further configured to determine a guidance signal based on the transducer pose and the target pose, such that the guidance signal represents a guidance for moving and/or rotating the ultrasonic handheld transducer from the transducer pose to the target pose.

According to an example, a camera is provided that is configured to at least indirectly acquire an outline image of a surface outline of a human subject.

According to an example, the camera unit is configured to acquire a track-image of the ultrasonic handheld transducer and a surrounding region of the surface of the human subject surrounding the ultrasonic handheld transducer, when the ultrasonic handheld transducer is arranged on the surface of the human subject.

As an effect, the guidance signal provides the basis for improving the actual pose of the ultrasonic handheld transducer, such that an operator of the ultrasonic handheld transducer can move and/or re-orient the ultrasonic handheld transducer accordingly, such that a match of the transducer pose and the target pose can be achieved. For instance, the respective guidance may be optically illustrated via and/or based on the guidance signal. As a result, an operator of the handheld transducer may receive an instruction how to move and/or to rotate the ultrasonic handheld transducer, such that the transducer pose will match the desired target pose. As a result, an improved ultrasonic image may be acquired via the ultrasonic handheld transducer. As a further result, an improved medical investigation and/or an improved medical diagnostic may be achieved.

In an example, the device and the ultrasonic handheld transducer may each be individually and/or separately formed. In a further example, the ultrasonic handheld transducer is not mechanically connected to the device. Thus, the device and the ultrasonic handheld transducer may be mechanically independent. Therefore, the camera unit of the device may acquire the track-image of the handheld transducer and a surrounding region of the surface of the human subject surrounding the ultrasonic handheld transducer. In an example, the device may be a mobile device, for instance a mobile phone or a mobile tablet computer device. In an example, the camera unit may comprise a 3D camera. The 3D camera may be configured to directly acquire the outline image as a 3D-image or as a depth image. In a further example, the camera unit may be a 2D camera. The camera unit may be configured to acquire a plurality of images, in particular 2D images, of the surface of the human subject and to determine the outline image, in particular as a 3D image or a depth image, based on the plurality of previously acquired images. In an example, the camera unit may comprise a sub-processing unit or may be configured to access the processing unit for determining the outline image. In a further example, the camera unit may comprise or maybe formed by a stereo camera, a time-of-flight camera, a laser range scanner, a structural light camera, a thermal sensor camera, or the like.

The processing unit is configured to access the human reference model. In an example, the human reference model may be stored in a memory unit of the device. The memory unit may be comprised by the device. Alternatively and/or additionally, the device may comprise an interface, in particular a communication interface, which is configured to access the human reference model from another device and/or system. The communication interface may be a radio communication interface or a cable communication interface.

The human reference model represents a surface outline of a virtual human subject. The virtual human subject does not necessarily represent an actual human subject. Instead, the virtual human subject may just statistically represent an arbitrary human subject or a collection of arbitrary human subjects.

The human reference model further represents the internal morphology of the virtual human subject and a relation between the surface outline of the virtual human subject and the internal morphology of the virtual human subject. In an example, the internal morphology of the virtual human subject may relate to and/or maybe an anatomical structure of the virtual human subject. An anatomical structure may relate to at least one inner organ, at least a part of a skeleton and/or at least a part of a vascular system. Thus, the human reference model may represent at least one inner organ of the virtual human subject, a surface outline of the virtual human subject and a relation between said surface outline and the at least one inner organ of the virtual human subject. The relation between the inner morphology and the surface outline may relate to a functional relation. Thus, the relation may represent the information how to amend or adapt the internal morphology, if the surface outline is subject to an amendment or adaptation, respectively. In other words, if an adaptation of the surface outline of the virtual human subject is to be carried out, the relation may provide the information how to correspondingly adapt the internal morphology. Preferably, the adaptation of the human reference model is performed such the adaptation is performed to the whole human reference model. As an effect, an adaptation of the surface outline of the human reference model would result in an analogous adaptation of the internal morphology of the human reference model, in particular based on the relation of said outline surface and said internal morphology. In an example, the processing unit may therefore be configured to perform the adaptation of the human reference model with respect to the whole human reference model resulting in the adapted model, which represents the fitted and/or adapted surface outline, a correspondingly adapted internal morphology and a correspondingly adapted relation between the fitted/adapted surface outline and the adapted internal morphology. In an example, the internal morphology of the adapted model refers to the internal morphology, which is represented by the adapted model. In a further example, the surface outline of the adapted model refers to the surface outline, which is represented by the adapted model. Thus, the human reference model may be adapted such that the resulting adapted model would match to an arbitrary human subject, in particular the human subject for which the outline image has been previously acquired.

In an example, the ultrasonic handheld transducer comprises a probe, which may be configured to emit and/or to receive ultrasonic radiation. As a result, the probe of the ultrasonic handheld transducer may be configured to acquire ultrasonic radiation from a field of view, where ultrasonic radiation has been previously emitted to and/or reflected from. As a further result, a detection range may be associated with the probe of the ultrasonic handheld transducer. In other word, the detection range may be the range, where an ultrasonic examination or scan may be performed with respect to the probe of the ultrasonic handheld transducer.

The processing unit is configured to access a transducer model. The transducer model may be stored in a memory of the device. Alternatively and/or additionally, the transducer model may be accessed via an interface (in particular a communication interface) of the device, in particular from a server and/or from the ultrasonic handheld transducer. For this purpose, a temporary signal connection may be provided between the device and the ultrasonic handheld transducer, for example via an USB-connection and/or via a radio connection. Based on such a signal connection, the transducer model may be accessed via the processing unit, for example for the server or the ultrasonic handheld transducer, respectively.

The transducer model represents a surface outline of the ultrasonic handheld transducer and a detection range of the probe of the ultrasonic handheld transducer. Thus, the transducer model may provide the information about the surface outline of the ultrasonic handheld transducer, in particular for recognizing the ultrasonic handheld transducer in an image. The detection range is preferably fixed to the probe of the ultrasonic handheld transducer. Thus, if the ultrasonic handheld transducer is recognized resulting in a position and/or orientation of the ultrasonic handheld transducer, based on this information, the position and/or orientation of the detection range of the recognized ultrasonic handheld transducer may be determined. Furthermore, the ultrasonic handheld transducer may be configured to scan a particular region of a human subject or to scan a particular inner organ of the subject. For example, the ultrasonic handheld transducer may be configured to scan a liver or a prostate of a human subject. The respective configuration may also relate to the detection range. For example, depending whether the liver or the prostate should be imaged, the detection rage may be different. As an effect, depending on the target scan region to be acquired, the respective information about the correspondingly configured detection range may be represented by the transducer model. In a further example, the processing unit may be configured to select or to adapt the detection range associated with the ultrasonic handheld transducer based on the target region to be scanned. Thus, the transducer model may represent an individual detection range of the probe of the ultrasonic handheld transducer or may represent an adaptable detection range of the probe of the ultrasonic handheld transducer.

For acquiring an ultrasonic image, the ultrasonic handheld transducer maybe arranged on the surface of a human subject. If this is the case, the camera unit is configured to acquire a track-image of the ultrasonic handheld transducer and a surrounding region of the surface of the human subject surrounding the ultrasonic handheld transducer. The track-image may be a two-dimensional image or a three-dimensional image. Since the track-image represents a surrounding region of the surface of the human subject, the track-image may also represent a sub-part of the previously acquired outline image of the surface outline of the human subject. As a result, the processing unit may be configured to perform a registration of the track-image with respect to the outline image, in order to determine temporary position and/or orientation information. This temporary position and/or orientation information may be used in order to determine a position and/or orientation of the ultrasonic handheld transducer arranged on the surface of the human subject via the processing unit.

Thus, the processing unit is configured to recognize the ultrasonic handheld transducer in the track-image based on the transducer model deriving in a transducer pose of the ultrasonic handheld transducer with respect to the human subject. In an example, the transducer pose may represent a transducer position of the ultrasonic handheld transducer with respect to the human subject and/or a transducer orientation of the ultrasonic handheld transducer with respect to the human subject. Thus, by using the outline image, the human reference model, the transducer model and the track-image, the processing unit may determine the actual transducer pose of the ultrasonic handheld transducer.

The processing unit is configured to receive a target signal representing at least indirectly the scan region of the internal morphology of the adapted model. In an example, the target signal may result from a user input to a user interface of the device. For example, the device may comprise a user input interface. Based on an user input via the user input interface, the user may select an inner organ represented by the internal morphology of the adapted model. The respectively selected inner organ may be the region to be scanned and thus may represent the scan region of the internal morphology of the adapted model. The target signal may be determined based on the user input. Thus, the target signal may represent at least indirectly the scan region of the internal morphology of the adapted model. As an effect, the target signal may comprise the information, where the human subject has to be scanned via the ultrasonic handheld transducer. In a further example, the processing unit may be configured to receive the target signal by accessing another device, for example a server via an interface of the device.

The processing unit is configured to determine a target pose for the ultrasonic handheld transducer with respect to the human subject based on the target signal, the transducer model and the adapted model, such that a virtual match of the detection range and the scan region would result. Thus, when the ultrasonic handheld transducer would be positioned and/or oriented in the target pose with respect to the human subject, the detection range of the probe of the ultrasonic handheld transducer would scan the correspondingly desired scan region of the human subject. In an example, when the ultrasonic handheld transducer is arranged at the target pose, for example a desired inner organ of the human subject of interest could be reliably scanned via the ultrasonic handheld transducer.

The processing unit is configured to determine a guiding signal based on the transducer pose and the target pose, such that the guidance signal represents a guidance for moving and/or rotating and/or orienting the ultrasonic handheld transducer from the transducer pose to the target pose. Thus, the guidance signal may provide the information how to move, rotate and/or orient the ultrasonic handheld transducer, such that the ultrasonic handheld transducer would be arranged in the target pose, where the ultrasonic handheld transducer is positioned and/or oriented in order to reliably scan the desired scan region of the human subject, in particular where an inner organ of the human subject of interest is arranged. As an effect, the ultrasonic handheld transducer arranged at the target pose may provide a signal representing an ultrasonic image of the inner organ of interest, which would enhance a subsequent medical diagnostic. Furthermore, the respective medical diagnostic may be of a higher reliability.

As an even further effect, based on the guidance signal, an operator with even less handling experience with respect to the ultrasonic handheld transducer maybe enabled to acquire a desired and reliable ultrasonic image of a desired region of human subject with the ultrasonic handheld transducer.

As an even further effect, the device may be configured to access an arbitrary transducer model of a respective arbitrary ultrasonic handheld transducer. Thus, the device may provide the further effect, that an operator of an arbitrary ultrasonic handheld transducer may receive a guidance in order to arrange said ultrasonic handheld transducer in a target pose in order to acquire a desired ultrasonic handheld image of high reliability.

According to an exemplary embodiment of the device, the human reference model comprises deformation data representing a relation between a deformation of the surface outline of the virtual human subject and a resulting deformation of the internal morphology of the virtual human subject, wherein the processing unit is configured to perform the adaptation of the human reference model based on the deformation data. In an example, the deformation data may be statistical data. Thus, the deformation data may be determined on data previously captured of a plurality of human subjects with different physical properties. The human reference model may therefore be a statistical model. The deformation data may represent the surface outline of each of a plurality of virtual human subjects and each of their internal morphology. The deformation data may further relate to information of a functional relation between each of the surface outlines and their respective internal morphology relating thereto. Thus, the deformation data may represent the information, how the internal morphology of a virtual human subject is to be deformed and/or adapted in response to a deformation applied to the outline surface of said virtual human subject (which may result during the adaptation process) such that the outline surfaces of the virtual and (real) human subject fit to each other. The resulting adapted model may therefore be determined also based on the deformation data.

According to a further exemplary embodiment of the device, the device comprises a display, wherein the device is configured to illustrate at least one graphical element via the display based on the guidance signal, such that the at least one graphical element indicates the guidance for moving and/or rotating the ultrasonic handheld transducer from the transducer pose to the target pose. In an example, the graphical element may refer to an arrow, a sign, and/or any other character and/or mark. The at least one graphical element may be formed and/or designed, such that illustrating such a graphical element would provide the information to an operator of the ultrasonic handheld transducer how to move and/or rotate the ultrasonic handheld transducer in order to reach the target pose for the ultrasonic handheld transducer. Thus, the operator may receive a guidance for moving and/or rotating the ultrasonic handheld transducer in order to scan a desired region of the human subject, in particular a desired inner organ of the human subject. As a result, an operator following the guidance provided by the at least one graphical element may be enabled to scan a desired scan region of the human subject, in particular a desired inner organ, via the ultrasonic handheld transducer with a high reliability. As an effect, an experienced or even an unexperienced operator of the ultrasonic handheld transducer may be enabled to scan said desired scan region of the human subject.

In an example, the device may be a mobile phone or a mobile tablet computer. Therefore, the device may comprise the display as well as the camera unit. In an example, the camera unit may be arranged at an opposite outside with respect to the display. Thus, the device may be configured to acquire at least one track-image and to illustrate the at least one graphical element in a real-time fashion. As an effect, the handling of the device may be enhanced.

According to a further exemplary embodiment of the device, the device comprises an optical projector, in particular a laser beam projector, wherein the device is configured to illustrate at least one graphical element via the optical projector on the surface of the human subject based on the guidance signal, such that the at least one graphical element indicates the guidance for moving and/or rotating the ultrasonic handheld transducer from the transducer pose to the target pose. In an example, the optical projector may be referred to a projector for projecting light or to a light projector. In an example, the graphical element may refer to an arrow, a sign, and/or any other marking element. In a further example, the at least one graphical element may be projected on the surface of the human subject next to the ultrasonic handheld transducer, such that an operator of the device and/or ultrasonic handheld transducer may receive the respective guidance in close vicinity to the ultrasonic handheld transducer. As an effect, the operator of the device and/or the ultrasonic handheld transducer does not have to interrupt the handling of the device and/or the ultrasonic handheld transducer, respectively. For example, if the operator is operating the ultrasonic handheld transducer and observing the ultrasonic handheld transducer being arranged on the surface of the human subject, the operator will recognize during same observation the at least one graphical element projected on the surface of the human subject, such that the operator can follow the guidance in order to arrange the ultrasonic handheld transducer at the target pose. As an effect, the ultrasonic handheld transducer being arranged in the target pose will allow an acquisition of ultrasonic image of the scan region of the human subject, in particular of a desired inner organ of the human subject. As a further effect, a reliable ultrasonic image may be acquired via the ultrasonic handheld transducer.

According to a further exemplary embodiment of the device, the device is configured to perform at least one update. For each update, (1.) the input unit is configured to receive an acquired further track-image (for example acquired by a camera) of the ultrasonic handheld transducer and of the surrounding region of the surface of the human subject surrounding the ultrasonic handheld transducer as an updated track-image, when the ultrasonic handheld transducer is arranged on the surface of the human subject, and (2.) the processing unit is configured to recognize the ultrasonic handheld transducer in the updated track-image based on the transducer model deriving in the updated transducer pose of the ultrasonic handheld transducer with respect to the human subject. The processing unit may further be configured to update at least once the guidance signal, in particular at the end of each update, based on the target pose and the previously updated transducer pose. As an effect, the guidance signal is updated after each update of the further/new track-image. For example, if an operator has improved the pose of the ultrasonic handheld transducer based on the illustrated graphical element indicating the respective guidance, a subsequent acquired track-image may be the "further track-image". Thus, an update of the guidance signal may be performed. Further, the updated guidance signal may be brought to the attention of the operator via a respective updated graphical element. In an example, the device is configured to illustrate at least one updated graphical element via the display based on the updated guidance signal. As a result, the operator may receive a feedback in order to subsequently improve the pose of the ultrasonic handheld transducer, in particular until the ultrasonic handheld transducer is arranged in the target pose. In an example, the device may be configured to illustrate a special graphical element as a graphical element via the display, when the ultrasonic handheld transducer has reached the target pose. As a result, the operator will receive the information, that the current pose of the ultrasonic handheld transducer may enable the ultrasonic handheld transducer to acquire a reliable and/or desired ultrasonic image of the scan region of the human subject, in particular of a desired inner organ of the human subject.

According to a further exemplary embodiment of the device, the device is configured to access a transducer dataset comprising a plurality of different transducer basic-models, each representing a surface outline of an associated ultrasonic handheld transducer and a detection range of its probe, wherein the device is configured to receive a transducer selection signal, which indicates one of the plurality of different transducer basic-models, and wherein the device is configured to select the transducer basic-model, which is indicated by the transducer selection signal, as the transducer model. In an example, the transducer dataset may refer to a dataset as such. In an example, the transducer selection signal may refer to a signal as such. In an example, the device may comprise an interface configured to receive the transducer selection signal. The interface may be a communication interface. Alternatively and/or additionally, the device may comprise a user input interface, which is configured to receive the transducer selection signal at least indirectly. In a further example, the device may comprise a memory, which stores the transducer dataset. Alternatively and/or additionally, the device may be configured to access the transducer dataset from a different device, in particular from a server. For this purpose, the device may comprise in particular a further interface configured to establish a signal connection to the further device, in particular the server, in order to access the transducer dataset. In an example, the different transducer basic-models may relate to different kinds and/or types of ultrasonic handheld transducers.

As an effect, the device may be configured to cooperate with different kinds and/or types of ultrasonic handheld transducers. In an example, depending on the type and/or model of the ultrasonic handheld transducers being used in the particular instance to be arranged on the surface of the human subject in order to acquire an ultrasonic image, the transducer selection signal may be used to select the respective transducer basic-model as the transducer model. As a result, the respective ultrasonic handheld transducer can be recognized in the track-image. As a further effect, a reliable transducer pose of the ultrasonic handheld transducer with respect to the human subject may be determined. As an effect, the transducer model to be used for a certain case may be selected via the transducer selection signal.

According to a further exemplary embodiment of the device, the device comprises an input panel, which may be formed by a or the display of the device, wherein the input panel and the processing unit are configured to determine the transducer selection signal based on an external operation of the input panel. In an example, the display may be a touch screen. Thus, the touch screen may form the display as such and may also form the input panel. The input panel may also be referred to as an interface of the device or a user input interface of the device.

In an example, the external operation of the input panel may refer to a touch operation and/or user input on the display at a certain surface area of the display, where the transducer basic-models to be selected are indicated. Thus, an operator may select via the input panel one of the indicated basic-models, such that the respective selection may result at least indirectly in the transducer selection signal. In an example, the display may illustrate a list of transducer basic-models, wherein the operator selects one item of said list resulting in a selection of a respective transducer basic-model resulting in a respective transducer selection signal, such that the selected transducer basic-model may form the transducer model. This transducer model may thereafter be used for instance for the recognition of the ultrasonic handheld transducer in the track-image. As an effect, the device may be configured to cooperate with different kinds and/or types of ultrasonic handheld transducers and/or with respective transducer models.

In an example, the device may be configured to control the display, such that at least a subset of the plurality of the transducer basic-models is indicated on the display. In an example, a list of the subset of the transducer basic-models or a list of all the transducer basic-models are indicated on the display. Thus, an operator may select one of the indicated transducer basic-models by touching the respective item of the list. Based thereon, the display and/or the processing unit may be configured to determine the respective transducer selection signal.

According to a further exemplary embodiment of the device, the device comprises an input interface, which is configured to receive a transducer model signal, which represents a transducer model. In an example, the processing unit may be configured via the input interface to access, preferably to receive, the transducer model signal and thus the respective transducer model. In an example, the input interface may be formed by a cable port. Thus, the device may be connected via the cable port to another device, for instance the ultrasonic handheld transducer, in order to receive the transducer model signal. However, the cable port of the device may be connected to another device, for instance a server, in order to receive the transducer model signal. The cable port may be configured to releasably connect to the other device. For instance, the cable port may be connected temporarily to the other device. After receiving the transducer model signal, the cable port may be disconnected. In a further example, the input interface may be formed as a radio interface. The radio interface may be configured to establish a signal connection to another device, for instance a server or the ultrasonic handheld transducer. As an effect, the transducer model maybe transmitted via the input interface and/or the respective transducer model signal to the processing unit. As a further effect, the device may be configured to cooperate with an arbitrary ultrasonic handheld transducer, since the respective transducer model may be received and/or accessed via the input interface.

According to a further exemplary embodiment of the device, the device comprises an input interface, which is configured to receive the transducer selection signal. In an example, the input interface may be the same input interface as described before or may be formed by another interface. In an example, the transducer selection signal may refer to a signal as such. In a further example, the transducer selection signal may represent identification information about a certain ultrasonic handheld transducer, for instance its model number and/or other data characterizing the respective ultrasonic handheld transducer. As a result, the processing unit may be configured to select the transducer model dependent on and/or based on the transducer selection signal. In an example, the transducer selection signal may be transmitted from a further device, in particular a server and/or the ultrasonic handheld transducer, to the input interface. Thus, the ultrasonic handheld transducer maybe configured to transmit the transducer selection signal to the input interface of the device. As a result, the ultrasonic handheld transducer being arranged on the surface of the human subject and being captured via the track-image may be recognized reliably. In other words, the transducer selection signal and the resulting transducer model may be used for the recognition purpose.

According to an exemplary embodiment of the device, the input interface is configured to establish a signal connection to the ultrasonic handheld transducer, such that the transducer model signal or the transducer selection signal is receivable from ultrasonic handheld transducer. As an effect, the device may receive the transducer model signal or the transducer selection signal from the ultrasonic handheld transducer. Further effects and/or results may be taken from the previous explanations in an analogous manner.

The input interface of the device may be formed by a radio interface or a cable port. The ultrasonic handheld device may also comprise an associated input interface, wherein this interface is referred to as a further input interface. The further interface of the ultrasonic handheld transducer maybe formed by a cable port or a radio interface. As an effect, the signal connection may be referred to a cable link connection or a radio link connection, respectively.

In an example, the device and/or the ultrasonic handheld transducer may be configured to establish the signal connection between the input interface of the device and the ultrasonic handheld transducer (in particular the further input interface of the ultrasonic handheld transducer) for a predefined time period, in particular a short time period. This time period may be sufficient in order to transmit the transducer model signal and/or the transducer selection signal. Thereafter, the signal connection may be interrupted and/or disconnected. As a result, the ultrasonic handheld transducer may be handled very flexible thereafter.

In an example, the ultrasonic handheld transducer may be connected, in particular for a short time period, to the input interface of the device in order to transmit its identification information, for instance its model number, via the transducer selection signal and/or to transmit the transducer model via the transducer model signal. The signal connection may be an USB-connection, a Bluetooth-connection or a wireless connection, in particular a wireless LAN-connection. Other signal connections may also be invisible.

As a further effect, the device may receive the transducer model signal or the transducer selection signal from an arbitrary ultrasonic handheld transducer. Thus, the device may be used and/or may be configured to cooperate with an arbitrary ultrasonic handheld transducer, in particular across all manufacturers of ultrasonic handheld transducers.

According to a further exemplary embodiment of the device, the device comprises an or the input interface, which is configured to receive an ultrasonic signal from the ultrasonic handheld transducer, wherein the ultrasonic signal represents an ultrasonic image, being acquired by the ultrasonic handheld transducer and illustrating a morphology-segment of the human subject, wherein the processing unit is configured to update the adapted model, such that the internal morphology represented by the updated adapted model fits to the morphology-segment of the human subject, wherein the processing unit is configured to update the target pose for the ultrasonic handheld transducer with respect to the human subject based on the target signal, the transducer model and the updated adapted model resulting in a virtual match of the detection range and the scan range, wherein the processing unit is configured to update the guidance signal based on the transducer pose and the updated target pose.

The ultrasonic signal may be referred to a signal as such.
As an effect, the device and in particular the processing unit may receive via the ultrasonic signal representing the ultrasonic image of an actual morphology-segment of the human subject further information of the actual human subject, in particular with respect to its morphology. This information therefore may be taken into account via the explained update in order to improve the updated model and/or to improve the target pose. As a result, the processing unit may update the guidance signal, in order to provide an operator of the ultrasonic handheld transducer a more improved guidance for moving and/or rotating the ultrasonic handheld transducer from the current transducer pose towards the target pose. In an example, the device may be configured to update the illustration of the at least one graphical limit via the display based on the updated guidance signal. Instead of the display, the optical projector maybe used.

In an example, the input interface of the device may be formed by a further input interface of the device configured to receive the ultrasonic signal. However, said input interface may alternatively be formed and/or integrated in the at least one input interface previously discussed with respect to the device.

According to a second aspect of the present invention, a system for providing a guidance for an ultrasonic handheld transducer is provided. The system comprises an ultrasonic handheld transducer, in particular as previously explained. The system further comprises a device according to the first aspect of the present invention and/or according to one of the previously explained embodiments and/or examples. The ultrasonic handheld transducer of the system is configured to be arranged on the surface of a human subject. The ultrasonic handheld transducer and the device are configured to transmit the guidance signal from the device to the ultrasonic handheld transducer. The ultrasonic handheld transducer comprises an output unit, in particular with optical means and/or acoustical means. The ultrasonic handheld transducer is configured to indicate the guidance via the output unit based on the guidance signal.

It is understood that, without repeating here all the explanations, examples, features, effects and/or advantages provided with reference to the device and or the ultrasonic handheld transducer, all the above examples, explanations, features, effects and/or advantages, may also to be intended as being provided in an analogous manner for the system.

As an effect, an operator of the ultrasonic handheld transducer may be guided where to arrange and/or how to orient the ultrasonic handheld transducer in order to scan a desired scan region of the human subject with a high reliability and/or with a high quality. In an example, at least one graphical guiding element may be illustrated via the output unit of the ultrasonic handheld transducer in order to illustrate the guidance for the operator of the ultrasonic handheld transducer.

In an example, the ultrasonic handheld transducer comprises optical and/or acoustical means for outputting the at least one graphical guiding element based on the guidance signal. In an example, the ultrasonic handheld transducer comprises a display for illustrating the at least one graphical guiding element, such as an arrows, based on the guidance signal.

As an effect, an operator of the ultrasonic handheld transducer may move and/or rotate the ultrasonic handheld transducer in accordance with the at least one graphical guiding element such that the actual transducer pose would match the target pose, even if the operator is not an expert with respect to the handling of the ultrasonic handheld transducer. As a result, a scan of the desired scan region of the human subject maybe achieved with high reliability.

According to a third aspect of the present invention, a method for determining a guidance signal is provided. The method comprises the steps of:
a) providing an at least indirectly acquired outline image of a surface outline a human subject, e.g. via a camera unit of a device;
b) accessing a human reference model, e.g. via a processing unit of the device, wherein the human reference model represents a surface outline of a virtual human subject, its internal morphology and a relation between its surface outline and its internal morphology;
c) adapting the human reference model, e.g. via the processing unit, resulting in an adapted model, such that the surface outline represented by the adapted model fits to the surface outline of the human subject;
d) accessing a transducer model, e.g. via the processing unit, wherein the transducer model represents a surface outline of a ultrasonic handheld transducer and a detection range of a probe the ultrasonic handheld transducer;
e) providing a track-image of the ultrasonic handheld transducer and a surrounding region of the surface of the human subject surrounding the ultrasonic handheld transducer, e.g. via the camera unit, when the ultrasonic handheld transducer is arranged on the surface of the human subject;
f) recognizing the ultrasonic handheld transducer in the track-image, e.g. via the processing unit and based on the transducer model resulting in a transducer pose of the ultrasonic handheld transducer with respect to the human subject;
g) receiving a target signal, e.g. via the processing unit, wherein the target signal represents at least indirectly a scan region of the internal morphology of the adapted model;
h) determining a target pose for the ultrasonic handheld transducer with respect to the human subject, e.g. via the processing unit and based on the target signal, the transducer model and the adapted model resulting in a virtual match of the detection range and the scan region; and
   i) determining a guidance signal, e.g. via the processing unit and based on the transducer pose and the target pose, such that the guidance signal represents a guidance for moving and/or rotating the ultrasonic handheld transducer from the transducer pose to the target pose.

With respect to the order of the steps it is noted the following: Step a) may be performed before step b) or before step c). Step b) may be performed before step c). Step c) may be performed before step d) or step e). Step d) may be performed before step e). Thus, step d) may even be performed before step a), b) or c). Step e) may be performed before step f). Step f) may be performed before step i). Step g) may be performed before step h). Step h) may be performed before step i).

It is understood that, without repeating here all the explanations, examples, features, effects and/or advantages provided with reference to the device and/or ultrasonic handheld transducer, the method of the present invention may be intended to be configured to carry out the method steps for which the device is configured to. Thus, all the above examples, explanations, features, effects and/or advantages, although previously provided with reference to the device and/or the ultrasonic handheld transducer, may also to be intended as being provided in an analogous manner for the method according to the third aspect of the present invention and/or to at least one of the following, exemplary embodiments of the method.

In an example, it is provided in step a): acquiring at least indirectly an outline image of a surface outline a human subject via a camera unit of a device;
In an example, it is provided in step e): acquiring a track-image of the ultrasonic handheld transducer and a surrounding region of the surface of the human subject surrounding the ultrasonic handheld transducer, e.g. via the camera unit, when the ultrasonic handheld transducer is arranged on the surface of the human subject.

According to an exemplary embodiment of the method, the method comprises the further step j.1) of: illustrating at least one graphical element via a display of the device based on the guidance signal, such that the at least one graphical element indicates the guidance for moving and/or rotating the ultrasonic handheld transducer from the transducer pose to the target pose.
According to a further exemplary embodiment of the method, the method comprises the further step j.2) of: illustrating at least one graphical element via a projector of the device on the surface of the human subject based on the guidance signal, such that the at least one graphical element indicates the guidance for moving and/or rotating the ultrasonic handheld transducer from the transducer pose to the target pose.

According to a further exemplary embodiment of the method, the method further comprises the steps:
k) performing at least one update; wherein for each update, a further track-image of the ultrasonic handheld transducer and of a surrounding region of the surface of the human subject surrounding the ultrasonic handheld transducer is acquired via the camera unit resulting in an updated track-image, when the ultrasonic handheld transducer is arranged on the surface of the human subject, and recognizing the ultrasonic handheld transducer in the updated track-image, e.g. via the processing unit based on the transducer model deriving in the updated transducer pose of the ultrasonic handheld transducer with respect to the human subject; and
l) updating at least once the guidance signal, in particular at the end of each update performed in step k), e.g. via the processing unit based on the target pose and an previously updated transducer pose.

According to a further exemplary embodiment of the method, wherein step d) comprises the sub-steps:
d.1) accessing, in particular, e.g. via the processing unit, a transducer dataset comprising a plurality of different transducer basic-models, each representing a surface outline of an associated ultrasonic handheld transducer and a detection range of its probe;
d.2) receiving, in particular, e.g. via the processing unit, a transducer selection signal, which indicates one of the plurality of the different transducer basic-models; and
d.3) selecting, in particular, e.g. via the processing unit, the transducer basic-model, which is indicated by the transducer selection signal, as the transducer model.

According to a further exemplary embodiment of the method, the method further comprises the steps:
m) acquiring an ultrasonic image of a morphology-segment of the human subject, e.g. via the ultrasonic handheld transducer;
n) transmitting an ultrasonic signal from the ultrasonic handheld transducer to the device, wherein the ultrasonic signal represents the ultrasonic image;
o) updating the adapted model, e.g. via the processing unit, such that the internal morphology represented by the updated adapted model fits to the morphology-segment of the human subject;
p) updating the target pose for the ultrasonic handheld transducer with respect to the human subject, e.g. via the processing unit and based on the target signal, the transducer model and the updated adapted model resulting in a virtual match of the detection range and the scan region; and
q) updating the guidance signal based on the transducer pose and the updated target pose, e.g. via the processing unit.

According to a further exemplary embodiment of the method, the method further comprises the step:
r) updating the illustration of the at least one graphical element based on the updated guidance signal.

According to a fourth aspect of the present invention, a computer program element is provided for controlling the device as explained above, which, when being executed by the processing unit, is adapted to carry out the method according to the present invention.

According to a fifth aspect of the present invention, a computer-readable medium having stored thereon a program element is provided, which, when being executed by the processing unit, is adapted to carry out the method of the present invention.

According to a further aspect of the present invention, a device for determining a guidance signal is provided. The device preferably relates to a mobile device, such as a mobile tablet computer. The device comprises a camera unit, a display and a processing unit. Via the camera unit, a three-dimensional outline image of a surface of a human subject is acquired. The acquiring of the three-dimensional outline image maybe performed by acquiring a plurality of two-dimensional pictures via the camera unit and thereafter determining the three-dimensional outline image based on the plurality of two-dimensional images. The determination of the three-dimensional outline image is performed by the processing unit. The display and the processing unit of the device may be configured to display the outline image of the surface of the human subject. As a result, an operator of the device may check whether the outline image illustrates a desired surface region of the human subject. The device further comprises a memory. The memory stores a human reference model. The human reference model may be previously accessed via an input interface of the device from a server or from an ultrasonic handheld transducer. For this purpose, the input interface may be configured and/or formed in order to establish a signal connection to the server or ultrasonic handheld device, respectively. The human reference model statistically represents a virtual human subject. In particular, the human reference model represents the virtual human subject by its surface outline, its internal morphology and a relation function between its surface outline and its internal morphology. The human subject model may be determined via a previous acquired data of different human subjects. The virtual human subject may for example represent an arithmetical average of the data previously acquired from the plurality of human subjects. Thus, the human reference model may function as a reference model. In practice it is often the case that the surface outline represented by the human reference model would not instantly fit to the surface outline of the human subject. Therefore, the processing unit is configured to adapt the human reference model resulting in an adapted model, such that the surface outline represented by the adapted model fits to the surface outline of the (real) human subject, from which the outline image has been previously acquired via the camera unit. Thus, the surface outline represented by the human reference model has to be deformed in order to fit the surface outline of the actual human subject. The respective deformation may at least be part of the adaptation, which the processing unit is configured to perform. Due to the adapted surface outline, which is now represented by the adapted model, an analogous adaptation of the internal morphology has to be performed in an analogous manner. Thus, the processing unit is configured to adapt the "whole" human reference model in order to receive the adapted model. In this context it is to be noted that the adaptation of the human reference model would also cover and/or relate to the adaptation of the internal morphology represented by the human reference model. This adaptation may be based on the functional relation between the surface outline and the internal morphology of the human reference model. Thus, the functional relation may provide the information how to adapt the internal morphology, if the surface outline represented by the human reference model is subject to an adaptation, in particular a deformation. The adapted model therefore represents a respectively adapted surface outline, a respectively adapted internal morphology and a relation between said surface outline and said internal morphology. In other words, the adapted model virtually represents the surface outline of the human subject and virtually represents the internal morphology of the human subject. Furthermore, the camera unit is configured to acquire a further image of the human subject, when said image is noted as a track-image. The image of the human subject is acquired, if the ultrasonic handheld transducer is arranged on the surface of this human subject. As a result, the track-image illustrates the ultrasonic handheld transducer in front of the surface of the human subject. In other words, the track-image illustrates the handheld transducer and a region of the surface of the human subject surrounding the ultrasonic handheld transducer. The processing unit is configured to recognize the ultrasonic handheld transducer in the track-image. For this purpose, the processing unit may access a transducer model. The transducer model may be stored in the memory of the device. Beforehand, the transducer model may be accessed via an input interface from a server or from the ultrasonic handheld transducer as such. The transducer model preferably represents a surface outline of the ultrasonic handheld transducer. Thus, the recognition of the ultrasonic handheld transducer may be performed based on the transducer model and the track-image. Moreover, the region surrounding the ultrasonic handheld transducer of the surface of the human subject illustrated in the track-image may be registered in the outline image of the human subject, such that the processing unit can determine based thereon the position and/or orientation of the ultrasonic handheld transducer with respect to the human subject. As a result, the processing unit is configured to determine a transducer pose (position and/or orientation) of the ultrasonic handheld transducer with respect to the human subject. In practice, the ultrasonic handheld transducer is to be arranged on the surface of the human subject in order to scan a desired scan region of the human subject. The scan region may relate to an inner organ of the human subject. Thus, an operator of the device and/or of the ultrasonic handheld transducer may select the scan region and/or the inner organ via the display of the device and/or an user input interface of the device. In an example, the display of the device may be a touch display. In this case, the internal morphology of the adapted model may be virtually illustrated via the display. Further, via the processing unit and the touch display, the device may be configured to receive a user input, for instance a touch operation at a position of the display illustrating an desired, in order to determine a target signal representing at least indirectly a scan region of the internal morphology of the adapted model. Based on the target signal (representing at least indirectly the desired scan region), the processing unit is configured to determine a target pose for the ultrasonic handheld transducer with respect to the human subject, such that an ultrasonic handheld transducer positioned and/or oriented in the target pose would be arranged to scan the desired scan region (in particular the desired inner organ) of the human subject. Thus, if an operator arranges the ultrasonic handheld transducer in the target pose, a desired and/or reliable ultrasonic image may be acquired from the scan region (in particular an inner organ) of the human subject. The processing unit of the device is therefore further configured to determine a guiding signal based on the target pose and the actual transducer pose of the ultrasonic handheld transducer. Often, the actual transducer pose is not the target pose resulting in a pose error. In order to reduce this pose error between the transducer pose and the target pose, the processing unit is configured to determine the guiding signal such that the guiding signal represents a guidance for moving and/or rotating the ultrasonic handheld transducer from the transducer pose to the target pose. For instance, the guidance signal may represent a directional arrow, a distance and/or an angle for which (each) the ultrasonic handheld transducer has to be moved and/or rotated, respectively, in order to reach the target pose from the transducer pose as a starting pose. For instance, said direction, distance and/or angle may be illustrated on the display of the device. Furthermore, guiding elements, such as arrows, may be displayed on the display of the device in order to illustrate for the operator how to change the position and/or orientation of the ultrasonic handheld transducer. Alternatively and/or additionally, a signal connection may be established between the device and the ultrasonic handheld transducer, such that the guidance signal can be transmitted to the ultrasonic handheld transducer. The ultrasonic handheld transducer may be configured to optically and/or acoustically output a signal or sign, in particular to illustrate at least one guidance elements, based on the guidance signal. For instance, the ultrasonic handheld transducer may comprise lightning units, in particular in form of arrows, which are highlighted depending on the guidance signal. For instance, an LED arrow may be highlighted illustrating and/or indicating a suggested movement for the ultrasonic handheld transducer to the left, if the target pose is on the left-hand side to the actual transducer pose of the handheld transducer.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically illustrates a first embodiment of the device as well as a first example of the ultrasonic handheld transducer in a first position.
Fig. 2 schematically illustrates the first embodiment of the device as well as the first example of the ultrasonic handheld transducer in a second position.
Fig. 3 schematically illustrates a second embodiment of the device as well as the first example of the ultrasonic handheld transducer.
Fig 4 schematically shows an exemplarily illustration of a screen of a display of the device.
Fig. 5 schematically illustrates an embodiment of the system according to the present invention.
Fig. 6 schematically illustrates an embodiment of the method according to the present invention.

### DETAILED DESCRIPTION OF EMBODIMENTS

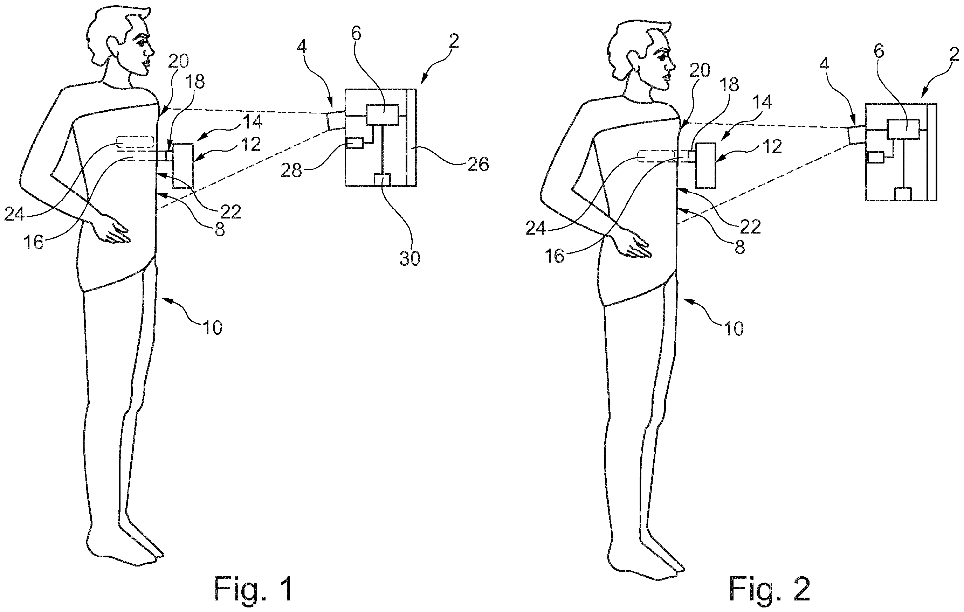

Fig. 1 schematically illustrates a device 2 for determining a guidance signal. Furthermore, Fig. 1 schematically illustrates a human subject 10 and an ultrasonic handheld transducer 14. The ultrasonic handheld transducer 14 is arranged on a surface 22 of the human subject 10. The probe 18 of the ultrasonic handheld transducer 14 is directed towards the human subject 10. In order to arrange the ultrasonic handheld transducer 14 on the surface 22 of the human subject, the ultrasonic handheld transducer 14 may be arranged directly or indirectly on the surface 22 of the human subject 10, in particular with its probe 18. The probe 18 of the ultrasonic handheld transducer is associated with a detection range 16. The detection range 16 is preferably the range, where ultrasonic radiation is provided by the probe 18 of the ultrasonic handheld transducer 14 and preferably reflected from the human subject 10, such that the reflected radiation may be detected by the probe 18 of the ultrasonic handheld transducer 14. Thus, the detection range 16 may relate to the range associated with the probe 18 of the ultrasonic handheld transducer 14, where an ultrasonic detection can be carried out.

The ultrasonic handheld transducer 14 is preferably formed by a mobile ultrasonic handheld transducer 14. The ultrasonic handheld transducer may therefore be cordless. Thus, it may be positioned and/or oriented very flexible with respect to the surface 22 of the human subject 10. As a result thereof, often only expert medical staff may perform an ultrasonic examination via the ultrasonic handheld transducer 14 with reliable ultrasonic image results. In order to overcome this drawback, the device 2 is preferably configured to determine a guidance signal representing a guidance for moving and/or rotating the ultrasonic handheld transducer 14, such that a desired scan region 24 of the human subject 10 can scanned reliably via the ultrasonic handheld transducer 14, in particular handled by an unexperienced operator.

The device comprises an input unit and a processing unit 6. Preferably, the device 2 further comprises a display 26. The input unit of the device 2 is configured to at to receive an at least indirectly acquired outline (8) image of a surface outline of a human subject. For example, a camera unit 4 is provided to least indirectly acquire the outline image of the surface 8 of the human subject 10. In particular in the case, where the device 2 is formed by a mobile tablet computer the camera unit 4 may comprise a two-dimensional camera. Via the 2D camera a plurality of two-dimensional images may be acquired of the surface 8 of the human subject 10. Based on these two-dimensional images, the camera unit 4, and in particular in combination with the processing unit 6, may determine an outlined image (preferably as a three-dimensional image or a depth image) of the surface outline 8 of the human subject 10. Thus, the indirect acquiring of the outline image of the surface outline 8 of the human subject 10 may comprise the acquiring of a plurality of two-dimensional images and the determination of the outline image based on said plurality of two-dimensional images. The camera unit 4, however, may alternatively and/or additionally comprise a three-dimensional camera. This three-dimensional camera may be configured to directly acquire a three-dimensional outline image of the surface outline 8 of the human subject 10.

The processing unit 6 is configured to access a human reference model. This model may refer to a data model or a human reference data model. The device 2 may comprise a memory unit 28. The human reference model may be stored by the memory unit 28. Thus, the processing unit 6 maybe configured to access the memory unit 28 in order to access the human reference model. Alternatively and/or additionally, the device 2 may comprise an interface 30. The interface 30 may also be referred to as an interface unit or a communication unit. The processing unit 6 may be configured to access the human reference model via the interface 30, in particular from a server or from the ultrasonic handheld device 14. For this purpose, a signal connection may be established between the interface 30 and the server or the ultrasonic handheld transducer 14, respectively. The signal connection may be a permanent signal connection or a temporarily established signal connection.

The human reference model represents a surface outline of a virtual human subject, its internal morphology and a relation between its surface outline and its internal morphology. Thus, the human reference model may serve as a reference model. It is desired to adapt the human reference model, such that the surface outline of the virtual human subject fits and/or matches to the previously acquired outline image of the surface outline 8 of the human subject 10. As a result of this fit and/or match procedure, the internal morphology represented by the human reference model would be adapted accordingly. In an example, the internal morphology represented by the human reference model represents at least one inner organ, at least a part of a skeleton and/or at least a part of a vascular system of the virtual human subject. Thus, the internal morphology represented by the human reference model may represent anatomical structures of the virtual human subject. Adapting the human reference model, such that surface outline of the adapted model fits to the surface outline of the human subject 10, would also result in an accordingly adapted internal morphology of the adapted model, such that the internal morphology of the adapted model would represent an estimation of the internal morphology of the human subject 10. In other words, the processing unit 6 is configured to adapt the human reference model resulting in the adapted model, such that the surface outline represented by the adapted model fits to the surface outline of the human subject 10. As discussed before, the adaptation of the human reference model resulting in the adapted model should be carried out to the whole human reference model. As a result, an analogous adaptation will be subject to the internal morphology. The adapted model may represent a surface outline (which fits to the surface outline of the human subject), an adapted internal morphology and a relation between the adapted surface outline and its adapted internal morphology. As an effect, the adapted model may provide a good estimation of a position and/or orientation of internal anatomical structures of the human subject 10, which are not visible from the outside or from the outline image of the surface outline 8 of the human subject 10. For example, the adapted model may encode a typical statistical relation between the surface outline 8 of the human subject 10 and the location and/or shape of internal organs. Therefore, the adapted model may provide the basis to provide a possible prediction of a shape and/or location of an internal organ.

The camera unit 4 is further configured to acquire a track-image of the ultrasonic handheld transducer 14 and a surrounding region 20 of the surface 22 of the human subject 10 surrounding the ultrasonic handheld transducer 14, when the ultrasonic handheld transducer 14 is arranged on the surface 22 of the human subject 10. In this context, it may be noted that the outline image of the surface outline 8 of the human subject 10 (previously acquired) preferably represents the surface outline 8 of the human subject 10 without the ultrasonic handheld transducer 14 being arranged on the surface 22 of the human subject 10.

Further, the processing unit 6 is configured to access a transducer model. The transducer model may be a data model. The transducer model may be stored on the memory unit 28 of the device 2. Alternatively or additionally, the transducer model may be accessed via the interface 30. Thus, the transducer model may be accessed via the interface 30 from a server or from the ultrasonic handheld transducer 14. With respect to the signal connection, all the respective explanations, examples and/or effects may also to be intended as being provided in an analogous manner for this signal connection.

The transducer model represents a surface outline 12 of the ultrasonic handheld transducer 14 and the detection range 16 of the probe 18 of the ultrasonic handheld transducer 14. Thus, the transducer model may provide the information to the processing unit 6 with respect to the surface outline 12 of the ultrasonic handheld transducer 14, which is illustrated in the track-image.

The processing unit 6 is therefore further configured to recognize the ultrasonic handheld transducer 14 in the track-image based on the transducer model deriving in a transducer pose of the ultrasonic handheld transducer 14 with respect to the human subject 10. The track-image illustrates the surrounding region 20 of the surface 22 of the human subject 10 surrounding the ultrasonic handheld transducer 14. Thus, a registration of said surrounding region 20 may be performed with respect to the outline image. Furthermore, the ultrasonic handheld transducer 14 maybe recognized in the track-image. Thus, the processing unit 6 may be configured based on these information to determine the transducer pose of the ultrasonic handheld transducer 14 with respect to the human subject 10. The transducer pose may relate to the position and/or orientation of the ultrasonic handheld transducer 14 with respect to the human subject 10.

Furthermore, the transducer model represents the detection range 16 of the probe 18 of the ultrasonic handheld transducer 14. With respect to Fig. 1, the processing unit 6 may be configured to determine which part of the human subject 10 is scanned via the ultrasonic handheld transducer 14 based on the transducer pose of the ultrasonic handheld transducer 14 and the detection range 16. As can be taken from picture 1, the detection range 16 maybe arranged below a desired scan region 24, where the human subject 10 should be scanned at. In order to determine, whether the detection range 16 matches with the desired scan region 24, the processing unit 6 may need information about the scan region 24.

The processing unit 6 is therefore configured to receive a target signal representing at least indirectly a scan region 24 of the internal morphology of the adapted model. Since the adapted model was previously determined in order to match with the human subject 10, the scan region 24 will very likely also represent the scan region of the internal morphology of the human subject 10. For simplicity reason, a match is assumed. Thus, the scan region 24 may refer to the human subject as well as to the adapted model. The target signal maybe provided to the processing unit 6 via the interface 30. Alternatively or additionally, the display 26 may be a touch screen. An operator may be able to provide an input signal via touching the touch screen to the processing unit 6, wherein the input signal represents the target signal or may provide the based to determine the target signal. For instance, the internal morphology of the adapted model may be at least partly displayed on the touch screen (display 26), such that an operator can select a particular inner organ. Based on this selection, the target signal may be determined via the processing unit 6.

The processing unit 6 is configured to determine a target pose for the ultrasonic handheld transducer 14 with respect to the human subject based on the target signal, the transducer model and the adapted model, such that a virtual match of the detection range 16 and the scan region 24 is achieved. Thus, if an operator would arrange the ultrasonic handheld transducer 14 in the target pose, the detection range 16 of the ultrasonic handheld transducer 14 would match or capture the scan region 24 of the human subject. This is exemplarily illustrated in Fig. 2. However, the handheld transducer 14 is preferably held in the operator's hand. Thus, it is desired to provide an information to the operator how to change the position and/or orientation of the ultrasonic handheld transducer 14, such that the ultrasonic handheld transducer 14 would scan the desired scan region 24 of the human subject 10. For this purpose, the processing unit 6 is configured to determine a guidance signal based on the transducer pose and the target pose, such that the guidance signal represents a guidance for moving and/or rotating the ultrasonic handheld transducer 14 from the transducer pose to the target pose. In an example, the guidance signal may represent the guidance for moving the ultrasonic handheld transducer 14 in an arbitrary direction and/or for rotating the ultrasonic handheld transducer 14 about an arbitrary axis, in particular about one axis associated with the ultrasonic handheld transducer 14.

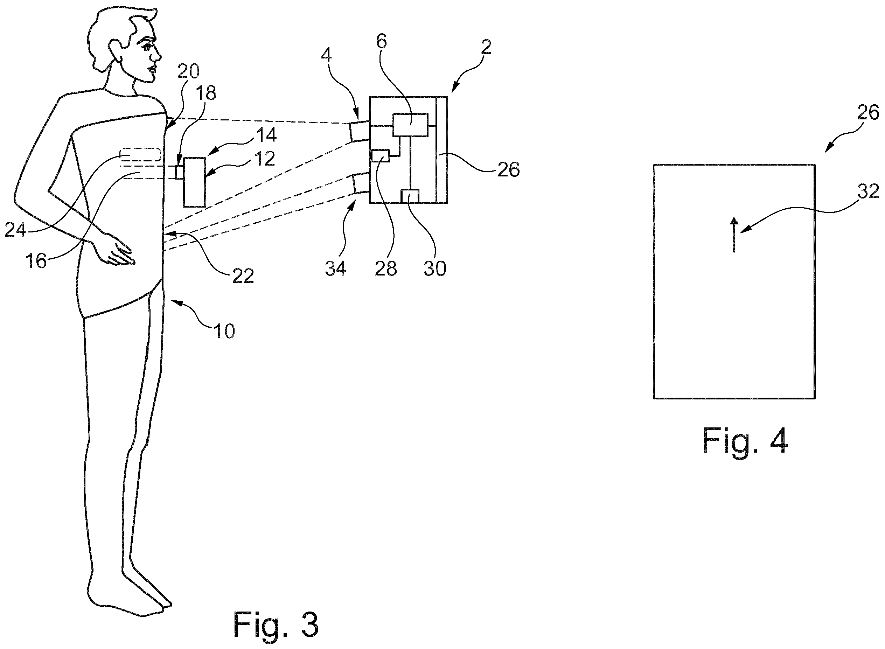

Fig. 4 exemplarily illustrates a screenshot of the display 26. The device 2 may be configured to illustrate at least one graphical element 32 via the display 26 based on the guidance signal, such that the at least one graphical element 32 indicates the guidance for moving and/or rotating the ultrasonic handheld transducer 14 from the transducer pose to the target pose.

From the synopsis of Fig. 1 and Fig. 2, it may be taken that the guidance signal should represent an upward movement of the ultrasonic handheld transducer 14. As a result, the device 2 may illustrate an arrow directed upwards via the display 26, as it is exemplarily shown in Fig. 4. Thus, an operator may receive the guidance to move the ultrasonic handheld transducer 14 upwards, in order to move it from the transducer pose shown in Fig. 1 towards the target pose for the ultrasonic handheld transducer 14, as it is exemplarily illustrated in Fig. 2. If the ultrasonic handheld transducer 14 reaches the target pose, a scan of the scan region 24 may be performed via the ultrasonic handheld transducer 14.

As can be exemplarily taken from Fig. 3, the device 2 may comprise a projector 34, in particular a laser beam projector. The device 2 maybe configured to optically illustrate at least one graphical element 32 via the projector 34 on the surface 22 of the human subject based on the guidance signal, such that the at least one graphical element 34 indicates the guidance for moving and/or rotating the ultrasonic handheld transducer 14 from the transducer pose to the target pose. The graphical element may be formed in an analogous manner to the graphical element 32 shown in Fig. 4. Thus, an arrow may be projected on the surface 22 via the projector 34. As an effect, an operator of the ultrasonic handheld transducer 14 may receive the information to move the ultrasonic handheld transducer 14 upwards in order to achieve a match of the actual transducer pose and the target pose.

Furthermore, the device 2 may comprise an acoustical output means, for instance a loudspeaker. The device 2 may be configured to output an acoustical signal, for instance a synthesized, predetermined speech, via the loudspeaker based on the guidance signal, such that the acoustical signal indicates the guidance for moving and/or rotating the ultrasonic handheld transducer from the transducer pose to the target pose.

With respect to the graphical element illustrated in Fig. 4, this graphical element 32 may give an operator of the ultrasonic handheld transducer 14 a rather qualitative information than a quantitative information. Thus, the operator may move the ultrasonic handheld transducer 14 upwards for a small distance. This distance may not be sufficient in order to achieve a match of the actual transducer pose and the target pose. Thus, it would be desired to provide the operator with a number of subsequent graphical elements 32 illustrated on the display 26 and/or via the projector 34, such that the operator can move and/or rotate the ultrasonic handheld transducer 14, until the desired match of the actual transducer pose and the target pose is achieved. Thus, the device 2 may be configured to update the guidance signal and/or the illustration of the graphical element 32.

In an example, the device 2 is configured to perform at least one update, wherein for each update, the camera unit 4 is configured to acquire a further track-image of the ultrasonic handheld transducer 14 and of a surrounding region 20 of the surface 22 of the human subject 10 surrounding the ultrasonic handheld transducer 14 as updated track-image, when the ultrasonic handheld transducer 14 is arranged on the surface 22 of the human subject, and wherein the processing unit 6 is configured to recognize the ultrasonic handheld transducer 14 in the updated track-image based on the transducer model deriving in the updated transducer pose of the ultrasonic handheld transducer 14 with respect to the human subject 10. The processing unit 6 may be further configured to update at least once the guidance signal based on the target pose and the previously updated transducer pose. After each update of the guidance signal, the device 2 may be configured to update the illustration of the at least one graphical element 32 based on the last updated guidance signal, such that the at least one graphical element 32 indicates the updated guidance for moving and/or rotating the ultrasonic handheld transducer from the updated transducer pose to the target pose.

In an example, the device 2 is configured to access a transducer dataset. The transducer dataset may also be referred to a dataset as such. The transducer dataset maybe stored on the memory unit 28 of the device 2. Alternatively or additionally, the transducer dataset may be accessed via the interface 30, in particular from a server. For this purpose, a permanent or temporary signal connection may be established via the interface 30 to the server. The transducer dataset comprises a plurality of different transducer basic-models. Each of the transducer basic-models may be formed in an analogous manner to the transducer model. However, the basic-models are different among each other. Thus, each of the transducer basic-models represents a surface outline of an associated ultrasonic handheld transducer 14 and a detection range 16 of its probe 18. Each of the transducer basic-models may therefore refer to another actual ultrasonic handheld transducer 14, in particular each of a different type.

The device 2 is further configured to receive a transducer selection signal. The transducer selection signal maybe a signal as such. The interface 30 of the device 2 may be configured to receive said transducer selection signal. Alternatively and/or additionally, the device 2 may comprise an input panel, which may be formed by the display 26 as a touch screen. Thus, the input panel and the processing unit 6 may be configured to determine the transducer selection signal based on an external operation of the input panel, for instance a touch at the touch screen. For example, the device 2 may be configured to control the display 26, such that at least a subset of the transducer basic-models is indicated on the display 26. An operator may select via a touch on the display 26 one of the items in the list and thus may select one of the transducers represented by the respective transducer basic-model indicated at list's item on the display 26. Based thereon, the processing unit 6 may be configured to determine the respective transducer selection signal. The processing unit 6 may be further configured to select the transducer basic-model, which is indicated by the transducer selection signal, as the transducer as such. As an effect, the device 2 may be configured to cooperate with each of a plurality of different kinds and/or types of ultrasonic handheld transducers 14. Further, the processing unit 6 may therefore be able to recognize an arbitrary ultrasonic handheld transducer 14 in the track-image, if the respective transducer model has been previously selected from the plurality of different transducer basic-models.

In particular in the case, where the transducer dataset comprising the plurality of different transducer basic-models is not stored on the memory unit 28, the device 2 maybe configured to receive the transducer model differently. For example, the device 2 may be configured to receive a transducer model signal via the interface 30, wherein the transducer model signal represents the transducer model. Thus, the transducer model signal may be transmitted to the device 2 beforehand in order to store the transducer model in the memory unit 28. Thereafter, the processing unit 6 may access the respective transducer model from the memory unit 28 in order to use the transducer model for instance for the recognition of the ultrasonic handheld transducer 14 in the track-image.

In an example, the input interface 30 of the device 2 is configured to establish a signal connection to the ultrasonic handheld transducer 14, such that the transducer model signal and/or the transducer selection signal is receivable from the ultrasonic handheld transducer 14. In an example, the ultrasonic handheld transducer 14 may comprise a (further) memory unit, which stores the transducer model corresponding to the respective ultrasonic handheld transducer 14. Thus, if the device 2 is to be used to cooperate with the respective ultrasonic handheld transducer 14, the signal connection may be at least temporarily established in order to transmit the transducer model signal to the device 2 via the interface 30, such that the transducer model can be stored in the memory unit 28 of the device 2. Similarly, the signal connection may be established in order to transmit the transducer selection signal to the device 2, wherein the transducer selection signal may represent information about the respective ultrasonic handheld transducer 14, for instance its model number and/or any other identification number. Thus, the device 2 may select the respective transducer basic-model based on the transmitted transducer selection signal, for instance indicating the model number of the respective ultrasonic handheld transducer 14, from the plurality of different transducer basic-models, such that the selected transducer basic-model will form the transducer model for the further purpose. Since the ultrasonic handheld transducer 14 may be generally configured to scan at least a part of the internal morphology of the human subject 10, the respective information may be used to improve the adapted model, in particular the internal morphology represented by the adapted model. Thus, an ultrasonic image acquired via the ultrasonic handheld transducer 14 may be transmitted to the device 2 in order to subsequently improve the adapted model, such that the adapted model would subsequently estimate the actual internal morphology of the human subject 10 more precisely.

In an example, the device 2 comprises the input interface 30, which is configured to receive an ultrasonic signal from the ultrasonic handheld transducer 14. The ultrasonic signal may be a signal as such. The ultrasonic signal may represent an ultrasonic image being acquired by the ultrasonic handheld transducer 14 and illustrating at least a morphology-segment of the human subject 10. The processing unit 6 of the device 2 may be configured to update the adapted model, such that internal morphology represented by the updated adapted model fits to the morphology-segment of the human subject 10. The processing unit 6 may further be configured to update the target pose for the ultrasonic handheld transducer 14 with respect to the human subject 10 based on the target signal, the transducer model and the updated adapted model resulting in a virtual match of the detection range and the scan region. The processing unit may further be configured to update the guidance signal based on the transducer pose and the updated target pose. Furthermore, the device 2 may be configured to update the illustration of the at least one graphical element.

As an effect, the at least one ultrasonic image acquired by the ultrasonic handheld transducer 14 may serve as a basis to perform an update of the target pose resulting in a subsequent update of the guidance signal. Further, the illustration of the at least one graphical element 32 may be updated to provide the operator of the ultrasonic handheld transducer 14 with the latest information how to move and/or rotate the ultrasonic handheld transducer 14 in order to achieve a match between the actual transducer pose and the updated target pose.

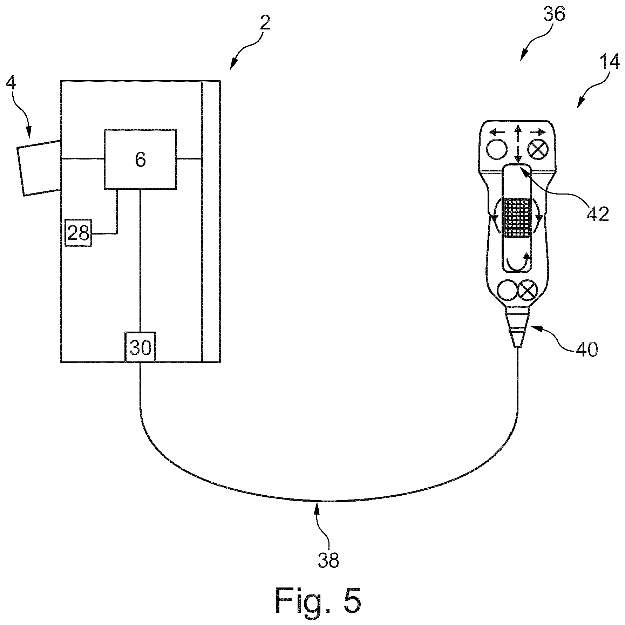

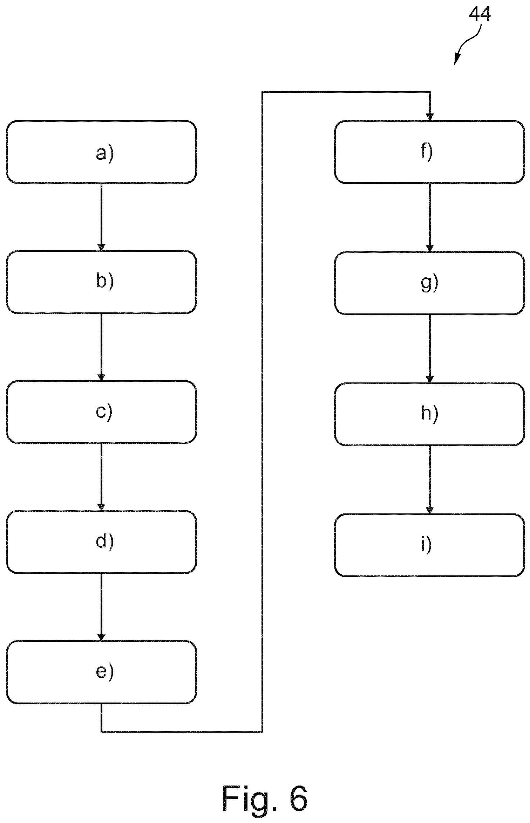

Fig. 5 schematically illustrates an example of the system 36 for providing a guidance for an ultrasonic handheld transducer 14. The system 36 comprises an ultrasonic handheld transducer 14 and a device 2. It is to be understood that, without repeating here all the examples, effects and/or explanations provided with reference to the device 2 as such and/or the ultrasonic handheld transducer 14 as such, the system 36 maybe intended as comprising the device 2 and/or the ultrasonic handheld transducer 14 as described above. Thus, all the above provided examples, explanations, effects and/or advantages, provided with reference to the device 2 and/or the ultrasonic handheld transducer 14, may also be intended as being implemented by the system 36. Thus, the ultrasonic handheld transducer 14 is configured to be arranged on the surface 22 of the human subject 10. The ultrasonic handheld transducer 14 and the device 2 are configured to transmit a guidance signal from the device 2 to the ultrasonic handheld transducer 14. For this purpose, a signal connection 38 maybe established between the device 2 and the ultrasonic handheld transducer 14. In an example, the signal connection 38 may be provided via a cable connection between the device 2 and the ultrasonic handheld transducer 14. The cable connection may be established between the interface 30 of the device 2 and a further interface 40 of the ultrasonic handheld transducer 14. Instead of a cable connection, a radio signal connection may be established between the interface 30 of the device 2 and the interface 40 of the ultrasonic handheld transducer 14. The ultrasonic handheld transducer comprises an output unit 42 in particular with optical means and/or acoustical means. The ultrasonic handheld transducer 14 is configured to indicate the guidance via the output unit 42 based on the guidance signal. Thus, the ultrasonic handheld transducer 14 may indicate via the output unit 42, in particular via the optical means, how to move and/or rotate the ultrasonic handheld transducer 14, such that an operator can achieve a match between the actual transducer pose and the target pose. Fig. 6 schematically illustrates an example of the method 44 for determining a guidance signal. The method 44 comprises the following:
In a first step a), an outline image of a surface outline of a human subject 10 is acquired at least indirectly via a camera unit 4 of a device 2.

In a second step b), a human reference model is accessed via a processing unit 6 of the device 2, wherein the human reference model represents a surface outline of a virtual human subject, its internal morphology and a relation between its surface outline and its internal morphology.

In a third step c), the human reference model is adapted via the processing unit 6 resulting in an adapted model, such that the surface outline represented by the adapted model fits to the surface outline of the human subject 10.

In a fourth step d), a transducer model is accessed via the processing unit 6, wherein the transducer model represents a surface outline of the ultrasonic handheld transducer 14 and a detection range of a probe 18 of the ultrasonic handheld transducer 14.

In a fifth step e), a track-image of the ultrasonic handheld transducer 14 and a surrounding region 20 of the surface 22 of the human subject 10 surrounding the ultrasonic handheld transducer 14 is acquired via the camera unit 4, when the ultrasonic handheld transducer 14 is arranged on the surface 22 of the human subject 10.

In a sixth step f), the ultrasonic handheld transducer 14 is recognized in the track-image via the processing unit 6 and based on the transducer model deriving in a transducer pose of the ultrasonic handheld transducer 14 with respect to the human subject.

In a seventh step g), a target signal is received via the processing unit 6, wherein the target signal represents at least indirectly a scan region 24 of the internal morphology of the adapted model.

In an eighth step h), a target pose of the ultrasonic handheld transducer 14 with respect to the human subject 10 is determined via the processing unit 6 and based on the target signal, the transducer model and the adapted model resulting in a virtual match of the detection range 16 and the scan region 24.

In a ninth step i), a guidance signal is determined via the processing unit 6 and based on the transducer pose and the target pose, such that the guidance signal represents a guidance for moving and/or rotating the ultrasonic handheld transducer 14 from the transducer pose to the target pose.

According to a further example of the present invention, a computer program element is provided, which, when being executed by a processing unit is adapted to carry out the method described above.

According to further example of the present invention, a computer readable medium having stored thereon a program element is provided, which, when being executed by a processing unit is adapted to carry out the method described above. In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention. This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to a device whereas other embodiments are described with reference to the method. However, a person skilled in the art will gather from the above that, unless otherwise notified, in addition to any combination of features belonging to one subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.
In the claims, the word "comprising" does not exclude other elements or steps, and the indefinite article "a" or "an" does not exclude a plurality. A single processing unit or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (2) for determining a guidance signal, the device comprising:
- an input unit; and
- a processing unit (6);
wherein the input unit is configured to receive an at least indirectly acquired outline (8) image of a surface outline of a human subject;
wherein the processing unit is configured to access a human reference model, which represents a surface outline of a virtual human subject, its internal morphology and a relation between its surface outline and its internal morphology;
wherein the processing unit is configured to adapt the human reference model resulting in an adapted model, such that the surface outline represented by the adapted model fits to the surface outline of the human subject;
wherein the processing unit is configured to access a transducer model, which represents a surface outline (12) of an ultrasonic handheld transducer (14) and a detection range (16) of a probe (18) of the ultrasonic handheld transducer;
wherein the input unit is configured to receive the image acquired as a track-image of the ultrasonic handheld transducer and a surrounding region (20) of the surface (22) of the human subject surrounding the ultrasonic handheld transducer, when the ultrasonic handheld transducer is arranged on the surface of the human subject;
wherein the processing unit is configured to recognize the ultrasonic handheld transducer in the track-image based on the transducer model deriving in a transducer pose of the ultrasonic handheld transducer with respect to the human subject;
wherein the processing unit is configured to receive a target signal representing at least indirectly a scan region (24) of the internal morphology of the adapted model;
wherein the processing unit is configured to determine a target pose for the ultrasonic handheld transducer with respect to the human subject based on the target signal, the transducer model and the adapted model resulting in a virtual match of the detection range and the scan region;
wherein the processing unit is configured to determine a guidance signal based on the transducer pose and the target pose, such that the guidance signal represents a guidance for moving and/or rotating the ultrasonic handheld transducer from the transducer pose to the target pose.

2. Device according to claim 1, wherein a camera unit (4) is configured to at least indirectly acquire the outline (8) image of the surface outline of the human subject; and
wherein, preferably, the camera unit is configured to acquire the track-image of the ultrasonic handheld transducer and the surrounding region (20) of the surface (22) of the human subject surrounding the ultrasonic handheld transducer, when the ultrasonic handheld transducer is arranged on the surface of the human subject.

3. Device according to one of the preceding claims, wherein the human reference model comprises deformation data representing a relation between a deformation of the surface outline of the virtual human subject and a resulting deformation of the internal morphology of the virtual human subject;
wherein the processing unit is configured to perform the adaptation of the human reference model based on the deformation data.

4. Device according to one of the preceding claims, wherein the device comprises:
i) a display (28), and wherein the device is configured to illustrate at least one graphical element (32) via the display based on the guidance signal, such that the at least one graphical element indicates the guidance for moving and/or rotating the ultrasonic handheld transducer from the transducer pose to the target pose; and/or
ii) an optical projector (34), in particular a laser beam projector, and wherein the device is configured to illustrate at least one graphical element via the optical projector on the surface of the human subject based on the guidance signal, such that the at least one graphical element indicates the guidance for moving and/or rotating the ultrasonic handheld transducer from the transducer pose to the target pose.

5. Device according to one of the preceding claims, wherein the device is configured to perform at least one update;
wherein for each update, the input unit is configured to receive an acquired further track-image of the ultrasonic handheld transducer and of a surrounding region of the surface of the human subject surrounding the ultrasonic handheld transducer as an updated track-image, when the ultrasonic handheld transducer is arranged on the surface of the human subject, and the processing unit is configured to recognize the ultrasonic handheld transducer in the updated track-image based on the transducer model deriving in the updated transducer pose of the ultrasonic handheld transducer with respect to the human subject; and
wherein the processing unit is configured to update at least once the guidance signal based on the target pose and an previously updated transducer pose.

6. Device according to one of the preceding claims, wherein the device is configured to access a transducer dataset comprising a plurality of different transducer basic-models, each representing a surface outline of an associated ultrasonic handheld transducer and a detection range of its probe;
wherein the device is configured to receive a transducer selection signal, which indicates one of the plurality of the different transducer basic-models; and
wherein the device is configured to select the transducer basic-model, which is indicated by the transducer selection signal, as the transducer model.

7. Device according to the preceding claim, wherein the device comprises an input panel, which may be formed by a or the display of the device; and
wherein the input panel and the processing unit are configured to determine the transducer selection signal based on an external operation of the input panel.

8. Device according to one of the preceding claims, wherein the device comprises an input interface (30), which is configured to receive a transducer model signal, which represents the transducer model.

9. Device according to one of the preceding claims, wherein the device comprises an input interface, which is configured to receive the transducer selection signal.

10. Device according to one of the preceding claims 8 to 9, wherein the input interface is configured to establish a signal connection to the ultrasonic handheld transducer, such that the transducer model signal or the transducer selection signal is receivable from the ultrasonic handheld transducer.

11. Device according to one of the preceding claims, wherein the device comprises an input interface, which is configured to receive an ultrasonic signal from the ultrasonic handheld transducer;
wherein the ultrasonic signal represents an ultrasonic image, being acquired by the ultrasonic handheld transducer and illustrating a morphology-segment of the human subject;
wherein the processing unit is configured to update the adapted model, such that the internal morphology represented by the updated adapted model fits to the morphology-segment of the human subject;
wherein the processing unit is configured to update the target pose for the ultrasonic handheld transducer with respect to the human subject based on the target signal, the transducer model and the updated adapted model resulting in a virtual match of the detection range and the scan region; and
wherein the processing unit is configured to update the guidance signal based on the transducer pose and the updated target pose.

12. A system (36) for providing a guidance for an ultrasonic handheld transducer (14), comprising:
- an ultrasonic handheld transducer (14); and
- a device (2) according to any of the preceding claims,
wherein the ultrasonic handheld transducer is configured to be arranged on the surface (22) of a human subject (10);
wherein the ultrasonic handheld transducer and the device are configured to transmit the guidance signal from the device to the ultrasonic handheld transducer,
wherein the ultrasonic handheld transducer comprises an output unit (42), in particular with optical means and/or acoustical means, and
wherein the ultrasonic handheld transducer is configured to indicate the guidance via the output unit based on the guidance signal.

13. A method (44) for determining a guidance signal, comprising the following steps:
a) providing an at least indirectly acquired outline image of a surface outline (8) a human subject (10);
b) accessing a human reference model, wherein the human reference model represents a surface outline of a virtual human subject, its internal morphology and a relation between its surface outline and its internal morphology;
c) adapting the human reference model resulting in an adapted model, such that the surface outline represented by the adapted model fits to the surface outline of the human subject;
d) accessing a transducer model, wherein the transducer model represents a surface outline (12) of a ultrasonic handheld transducer (14) and a detection range (16) of a probe (18) the ultrasonic handheld transducer;
e) providing a track-image of the ultrasonic handheld transducer and a surrounding region (20) of the surface (22) of the human subject surrounding the ultrasonic handheld transducer, when the ultrasonic handheld transducer is arranged on the surface of the human subject;
f) recognizing the ultrasonic handheld transducer in the track-image and based on the transducer model deriving in a transducer pose of the ultrasonic handheld transducer with respect to the human subject;
g) receiving a target signal, wherein the target signal represents at least indirectly a scan region (24) of the internal morphology of the adapted model;
h) determining a target pose for the ultrasonic handheld transducer with respect to the human subject and based on the target signal, the transducer model and the adapted model resulting in a virtual match of the detection range and the scan region; and
i) determining a guidance signal and based on the transducer pose and the target pose, such that the guidance signal represents a guidance for moving and/or rotating the ultrasonic handheld transducer from the transducer pose to the target pose.

14. A computer program element for controlling the device according to one of the claims 1 to 11, which, when being executed by a processing unit, is adapted to perform the method steps of claim 13.

15. A computer readable medium having stored the program element of claim 14.
